# EUROPEAN PATENT SPECIFICATION

(11) **EP 1 480 707 B1**
(45) Date of publication and mention of the grant of the patent: **26.07.2006**
(21) Application number: 02713691.0
(22) Date of filing: 27.02.2002
(51) Int. Cl.: A61M 29/00, A61M 5/32

(54) **AUTOMATICALLY RETRACTABLE NEEDLE SAFETY SYRINGE**
SICHERHEITSSPRITZE MIT AUTOMATISCH ZURÜCKZIEHBARER KANÜLE
SERINGUE DE SECURITE A AIGUILLE AUTOMATIQUEMENT RETRACTABLE

(43) Date of publication of application: 01.12.2004
(73) Proprietor: Safeshot Technologies, LLC, Mission Viejo CA 92691 (US)
(72) Inventor: Van Dyke, Lewis R., Gastonia, NC 28054 (US)
(74) Representative: Gervasi, Gemma
(86) International application number: PCT/US2002/005784
(87) International publication number: WO 2003/072181

(56) References cited:
- WO-A-97/10867
- DE-A1- 19 528 091
- US-A- 5 000 736
- US-A- 5 030 208
- US-A- 5 215 533
- US-A- 5 232 458
- US-A- 5 378 240
- US-B1- 6 402 721

## Description

### FIELD OF THE INVENTION

This invention relates generally to safety syringes, and more specifically to a safety syringe apparatus and method for automatically and immediately retracting a hollow needle permanently and protectively within the syringe body after a single use.

### BACKGROUND OF THE INVENTION

In recent years, the public has become increasingly aware of the health hazards associated with needle reuse and accidental needle prickings. This is true, especially among drug addicts, drug users (e.g., diabetics), medical personnel and healthcare providers. More than twenty blood-borne pathogens can be transmitted by the reuse of needles or accidental needle prickings, just a few of which include human immunodeficiency virus (HIV), acquired immunodeficiency syndrome (AIDS), hepatitis B, hepatitis C, syphilis, malaria, tuberculosis, and herpes.

The problem of spreading blood-borne pathogens through the reuse of needles is significant among drug addicts unwilling or unable to pay for sterile needles. The United States government, having recognized and acknowledged this problem, has attempted to control the reuse of syringe needles among drug addicts by establishing needle exchange programs where drug addicts can obtain free sterile needles in exchange for their contaminated needles. Despite this effort, at least 36% of HIV/AIDS cases and more than 50% of hepatitis B and hepatitis C cases in the United States can be linked to the sharing of needles among drug addicts. With approximately one million people with HIV/AIDS, more than 1.25 million hepatitis B carriers and more than 3.5 million hepatitis C carriers in the United States, the need to curb the practice of sharing needles is great. With more than 1.3 million injection drug users in the United States, the need for syringes having an integral, unremovable and unoverridable safety feature that limits the syringe to only a single use is overwhelming. (tri-ject.net/stats.html).

In addition, the spreading of blood-borne pathogens through the reuse of contaminated needles by drug addicts, drug users, medical personnel and healthcare providers in other countries throughout the world is becoming increasingly prominent. For example, approximately 30% of reported HIV/AIDS cases in Brazil, Chile, Uruguay, Paraguay and Argentina are directly related to the sharing of contaminated needles among drug addicts. Nearly 74% of injection drug addicts in Spain are HIV infected. Approximately 70% of the HIV cases reported in China are directly linked to the sharing of contaminated needles. In eastern European countries, 80% of injection drug addicts admit to sharing contaminated needles. Approximately 43% of the HIV/AIDS cases reported in Poland and Yugoslavia are linked to the sharing of contaminated needles among drug addicts. Furthermore, It is estimated that approximately 22 million people worldwide are living with HIV or AIDS. Unfortunately, in many countries, especially third world countries, sterile syringes are simply unavailable due to economic reasons. (www.vanishpoint.com/needlestick.html).

Although approximately one million accidental needle prickings are reported by healthcare workers annually, at least three million accidental needle prickings occur each year that subsequently go unreported. Various studies estimate that out of all the needle pricking injuries that occur to nurses, approximately 40% to 53% go unreported. Various studies also estimate that out of all the needle pricking injuries that occur to laboratory technicians, approximately 92% go unreported. Various studies further estimate that out of all the needle pricking injuries that occur to physicians, approximately 70% to 95% go unreported. (www.osha-slc.gov/SLTC/needlestick/safemeedle devices/saferneedledevices.html).

In 1997, the Centers for Disease Control and Prevention (CDC) sponsored a study which found that approximately 76% of needle pricking injuries could be avoided by using safety needles. As a result, needle legislation has now been introduced in approximately twenty-five states and in the District of Columbia. In fact, such safety needle legislation has already been signed into law in a number of states including California, Texas, Tennessee, New Jersey and Maryland. In addition, the Occupational Safety and Health Administration (OSHA) has promulgated a Blood-borne Pathogens Standard requiring employers to evaluate the effectiveness of existing controls designed to minimize or eliminate employee occupational exposure and to review the feasibility of instituting more advanced controls. Furthermore, the Food and Drug Administration (FDA), in an effort to protect health care workers, has set forth guidelines suggesting specific features that a safety syringe should possess. These include a safety feature that is not only simple and self evident to operate, thus requiring little or no additional training to use effectively, but also a safety feature that is an integral part of the apparatus. In other words, the guidelines suggest that the safety feature itself be unremovable and utilization of the safety feature be unavoidable. (www.osha-slc.gov/SLTC/needlestick/saferneedledevices/saferneedledevices.html; www.seiu.org).

As a result of the foregoing state legislation and agency guidelines, a great amount of time, effort and money has been invested by syringe manufacturers in developing syringes with safety needle designs. Presently, there are at least 250 types of safety syringes. However, the safety syringes that currently exist have been criticized for generally being too expensive to manufacture and having a safety feature that is not an integral part of the safety syringe. Another criticism includes safety syringes that are not economically feasible because operation of the safety feature is not self evident and therefore additional training is required to use the apparatus effectively. Additionally, the safety feature of at least one safety syringe is simply ineffective at preventing the transmission of blood-borne pathogens due to "reflux" blood contamination.

Of the current safety syringes, safety syringes using a spring mechanism are the most common for automatically retracting a hollow needle after injecting a fluid. However, these safety syringes are typically more expensive because of the required incorporation of additional materials for manufacture. Standard or conventional hypodermic needle syringes typically cost from five to seven cents each. On the other hand, the median increase in cost for a safety syringe is approximately thirty cents or more. At first glance, this minimal cost increase does not seem significant. However, after considering the thousands, if not millions, of needles used each year, the resultant increase in annual cost for utilizing the more expensive safety syringe is unfortunately excessive.

Another type of safety syringe is a syringe using a protective shield that slides and locks over the needle to protectively encase the piercing tip. However, the protective shield is not an integral part of the safety syringe. Because the protective shield slides manually over the needle, this safety feature may be overridden by a person who inadvertently or purposely fails to slide the shield over the needle thereby exposing a contaminated and potentially infectious piercing tip. Additionally, the protective shield requires two hands to slide the shield over the needle to encase the syringe needle. As a result, a person's hands may slip while sliding the shield and subsequently be pricked with the exposed and contaminated piercing tip.

Another type of safety syringe is a needleless jet injector that shoots a pinpoint jet of fluid through the skin at extremely high velocities. However, this safety syringe is not economically feasible because operation is not self evident and, therefore, costly time consuming training is required to use the apparatus effectively. Additionally, the needleless jet injector has been linked to causing hepatitis B infections resulting from "reflux" blood contamination of the injector heads from the previous injection. One approach is to provide proper cleaning and maintenance of the injector heads between injections, however this is time consuming and is not failsafe at preventing subsequent transmission of infectious blood-born pathogens. Another approach is to provide needleless jet injectors with nozzles and fluid chambers that can both be discarded after each injection. However, these safety features are mere accessories to, and are not built-in as integral parts of the apparatus. Therefore, the safety features of the needleless jet injector could be removed or even overridden by not utilizing a sterile, unused nozzle and/or fluid chamber attachments.

US5215533 discloses a safety syringe which includes a body, a plunger mounted within the body, connection means for connecting the plunger to a needle holder whereby subsequent retraction of the plunger withdraws the needle holder and the needle with a shielded position within the body effected by a vacuum chamber defined between the plunger and the syringe body, vacuum being created within the chamber by movement of the plunger during the injection stroke and serving to withdraw the plunger, the needle holder and needle after injection pressure is removed. The syringe may be provided with braking means.

Therefore a need exists for an effective and efficient, inexpensive safety syringe that is simple and self evident to operate and integrally comprises a safety feature having a hollow needle that protectively retracts automatically after 8 single injection. Further needed is a safety syringe that alleviates needle reuse and accidental needle prickings with contaminated syringe needles and therefore ultimately assists in preventing the transmission of blood-borne pathogens such as HIV/AIDS, hepatitis B and hepatitis C via contaminated syringe needles.

### OBJECTS OF THE INVENTION

The principal object of the present invention is to provide an automatically retractable needle safety syringe apparatus that injects fluid from a syringe and subsequently automatically and immediately retracts the piercing tip end of a hollow needle permanently within the safety syringe body, protectively pressed against the inner surface of the tube, after a single use thus preventing the transmission of blood-borne pathogens, such as HIV/AIDS, hepatitis B, hepatitis C and other diseases, via needle reuse and accidental needle prickings with contaminated syringe needles.

Another object of the present invention is to provide a method of automatically and immediately retracting the piercing tip end of a hollow needle permanently within the safety syringe body, protectively pressed against the inner surface of the tube, after a single use thus preventing the transmission of blood-home pathogens, such as HIV/AIDS, hepatitis B, hepatitis C and other diseases, via needle reuse and accidental needle prickings with contaminated syringe needles.

Another object of the present invention is to provide a safety syringe apparatus that permanently prevents needle reuse or accidental needle prickings with contaminated syringe needles by providing a protective barrier between the hands and the hollow needle automatically and immediately after a single use and during disposal.

Another object of the present invention is to provide an automatically and immediately retractable needle safety syringe apparatus that is both efficient and effective at preventing the transmission of blood-borne pathogens and other diseases through needle reuse or accidental needle prickings with contaminated syringe needles.

Another object of the present invention is to provide an automatically and immediately retractable needle safety syringe apparatus that is simple and inexpensive to manufacture, relative to the cost of manufacturing standard or conventional hypodermic needle syringes.

Another object of the present invention is to provide an automatically and immediately retractable needle safety syringe apparatus that is simple and self evident to operate and therefore requires no additional training to use the apparatus effectively and efficiently.

Another object of the present invention is to provide an automatically and immediately retractable needle safety syringe apparatus having a safety feature that is an integral part of the safety syringe, meaning that the safety feature is bnilt-in as a necessary, inherent and integral part of the apparatus and is therefore not merely an accessory which might be removed, circumvented or overridden.

### SUMMARY OF THE INVENTION

The present invention is a safety syringe apparatus according to claim 1 and method according to claim 14 for automatically and immediately retracting a hollow needle permanently and protectively within the syringe body after a single use. The present invention prevents needle reuse and accidental needle prickings with contaminated syringe needles.

In the preferred embodiment, the invented apparatus includes a syringe body having a plunger and a needle body, both of which are slidably disposed and partially positioned within the syringe body. The syringe body includes a shaft seal at a first end of the syringe body and a variable vacuum compartment. The plunger includes a shaft, a piston seal attached proximally to one end of the shaft and a punch located at the end of the shaft. The shaft of the plunger is slidably engageable with the shaft seal of the syringe body. The piston seal af the plunger is slidably disposed within the syringe body. The syringe body between the shaft seal and the piston seal defines the variable vacuum compartment. The needle body is temporarily attached to a second end, opposing the first end, of the syringe body and includes a piercing tip end attached to a ferrule.

During an injection stroke of a single injection of fluid, a vacuum is created within the variable vacuum compartment as a result of an increase in volume within the variable vacuum compartment without a corresponding influx of air molecules and fluid molecules into the variable vacuum compartment, due to the shaft seal and the piston seal being substantially air tight and fluid tight. At the end of the injection stroke, the punch frictionally engages the ferrule. The needle is immediately and automatically withdrawn within the syringe body as a result of the vacuum created within the variable vacuum compartment during the injection stroke. The needle body is withdrawn into the syringe body such that the piercing tip end permanently resides enclosed within and protectively pressed against the syringe body. This alleviates needle reuse and accidental needle prickings and, therefore, ultimately prevents the transmission of blood-borne pathogens and other diseases by contaminated syringe needles.

The invented safety syringe apparatus is efficient and effective at preventing the transmission of blood-borne pathogens and other diseases through needle reuse or accidental needle prickings with contaminated syringe needles. The safety syringe is also inexpensive to manufacture relative to the cost of manufacturing standard or conventional hypodermic needle syringes. In addition, the safety feature of the invented apparatus is simple and self evident to operate and therefore requires no additional training to use the apparatus efficiently and effectively.

This safety feature is an integral part of the invented safety syringe apparatus, meaning that the safety feature is built-in as a necessary, inherent and integral part of the apparatus. Because this safety feature is not a mere accessory which might be removed, circumvented or overridden after a single use, the piercing tip end permanently resides enclosed within and protectively pressed against, the syringe body therefore providing a protective barrier between the person's hands and the contaminated needle. Furthermore, this safety feature goes into effect automatically and immediately after a single use and remains in effect during disposal, thus alleviating needle reuse and accidental needle prickings with contaminated syringe needles. Therefore, the safety feature ultimately prevents the transmission of blood-borne pathogens such as HIV/AIDS, hepatitis B, hepatitis C and other diseases by needle reuse and accidental needle prickings with contaminated syringe needles.

### BRIEF DESCRIPTION OF THE DRAWINGS

The foregoing and other objects will become more readily apparent by referring to the following detailed description and the appended drawings in which:
FIG. 1 is a cross sectional view of a safety syringe apparatus in accordance with the present invention.
FIG. 2 is an exploded cross sectional view of the syringe body shown in FIG. 1.
FIG. 3 is an exploded cross sectional view of the plunger shown in FIG. 1.
FIG. 4 is a cross sectional view of the needle body shown in FIG. 1.
FIG. 5 is a detailed enlarged cross sectional view of a portion of the safety syringe apparatus shown in FIG. 1.
FIG. 6 is a cross sectional view of the needle body permanently residing enclosed within the syringe body and protectively pressed against the inner surface of the syringe body immediately after a single use in accordance with the present invention.

### DETAILED DESCRIPTION

The present invention is a safety syringe apparatus and method for automatically and immediately retracting a hollow needle permanently and protectively within the syringe body after a single use. The present invention prevents needle reuse and accidental needle prickings with contaminated syringe needles.

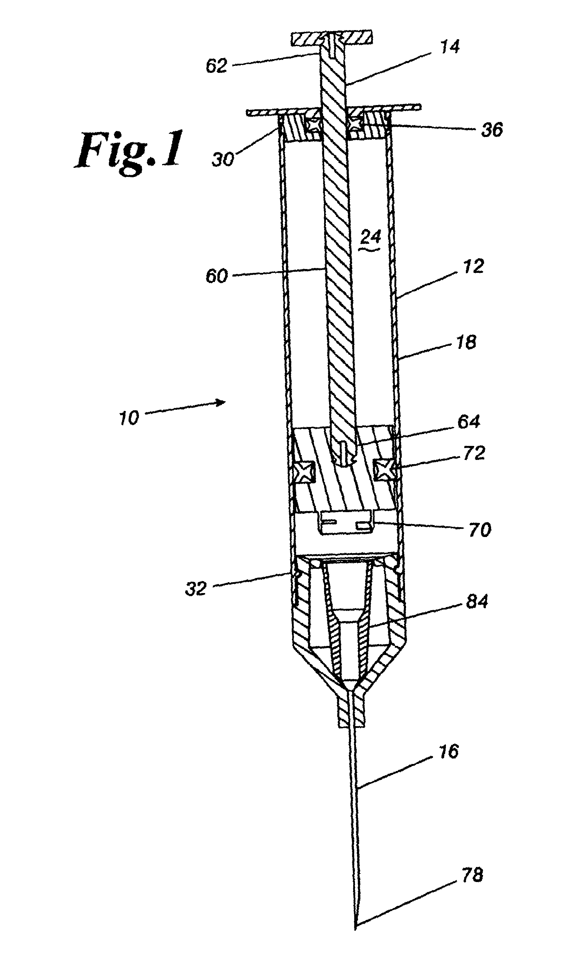

FIG. 1 is a cross sectional view of a safety syringe apparatus 10 in accordance with the present invention. An embodiment of the invented safety syringe apparatus 10, as shown in FIG. 1, includes a syringe body 12, a plunger 14 and a needle body 16. Both the plunger 14 and the needle body 16 are slidably disposed and partially positioned within the syringe body 12. The syringe body 12 is a tube 18 having two opposing ends 30, 32 and includes a shaft seal 36 and a variable vacuum compartment 24 at one end 30 of the syringe body 12. The plunger 14 includes a shaft 60 having a thumb end 62 and a piston end 64 opposing the thumb end 62, a piston seal 72 attached proximally to the piston end 64 of the shaft 60 and a punch 70 attached to the piston end 64 of the shaft 60. The shaft seal 36 and piston seal 72 maintain a substantially air tight and fluid tight seal between the shaft 60 and the syringe body 12 as the plunger 14 is displaced into and out of the syringe body 12. The syringe body 12 between the shaft seal 36 and the piston seal 72 defines the variable vacuum compartment 24. The needle body 16 is temporarily attached to a second end 32 of the syringe body and includes a piercing tip end 78 attached to a ferrule 84.

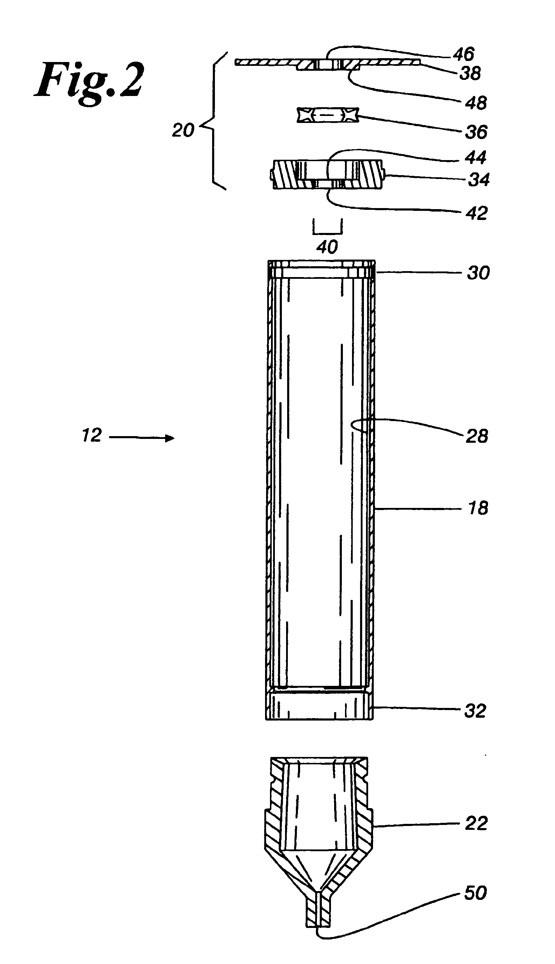

FIG. 2 is an exploded cross sectional view of the syringe body 12. The tube 18 includes an inner surface 28, a shaft seal attachment end 30 and a needle attachment end 32 opposing the shaft seal attachment end 30. The inner surface 28 of the tube 18 extends within and along the tube 18 between the shaft seal attachment end 30 and the needle attachment end 32. As shown in FIG. 2, the syringe body 12 additionally includes a shaft seal attachment 20 insertably attachable to the shaft seal attachment end 30 for receiving the previously mentioned plunger 14.

The shaft seal attachment 20 includes an attachment base 34, a shaft seal 36, an attachment head 38 and a shaft orifice 40 formed there through. The attachment base 34 has a shaft opening 42 for receiving the previously mentioned shaft 60 and a shaft seal annular groove 44 for receiving the shaft seal 36. The attachment head 38 is equipped with a shaft opening 46 and a shaft seal annular groove tab 48 that couples the attachment head 38 to the attachment base 34 so that the shaft seal 36 is interposed between the attachment head 38 and the attachment base 34. The shaft seal attachment 20 is assembled by coupling the attachment head 38 to the attachment base 34. Once assembled, the shaft orifice 40 of the shaft seal attachment 20 is formed by the shaft opening 42 of the attachment base 34, the shaft opening 46 of the attachment head 38 and the shaft seal 36. During assembly, the shaft seal attachment 20 is attached to the tube 18 by inserting the shaft seal attachment 20 into the shaft seal attachment end 30 of the tube 18.

The syringe body 12 additionally includes a needle attachment 22 insertably attachable to the needle attachment end 32 for receiving the previously mentioned needle body 16. The needle attachment 22 includes a needle receiving conduit 50. During assembly, the needle attachment 22 is attached to the tube 18 by inserting the needle attachment 22 into the needle attachment end 32 of the tube 18.

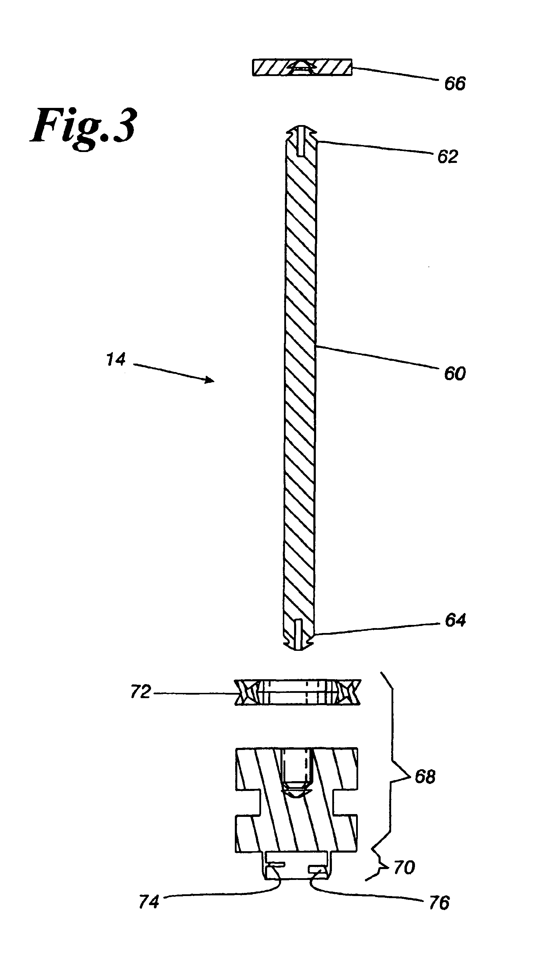

FIG. 3 is an exploded cross sectional view of the plunger 14 shown in FIG. 1. The plunger 14 includes the previously mentioned shaft 60, thumb end 62 and piston end 64 opposing the thumb end 62, and also includes a thumb platform 66 attached to the thumb end 62, a piston 68 attached to the piston end 64 and the previously mentioned punch 70 attached to the piston end 64 and positioned adjacent the piston 68. In an alternative embodiment, the punch 70 is attached to the piston 68 and is positioned adjacent the piston end 64 of the plunger 14. The piston 68 is equipped with a piston seal 72 attached proximally to the piston end 64 of the plunger 14. The shaft 60 is preferably a solid elongate cylindrical tube shape. In an alternative embodiment, the shaft 60 is substantially hollow to provide additional volume within the previously mentioned variable vacuum compartment 24 for creating a vacuum while maintaining requisite structural integrity of the shaft 60. In another alternative embodiment, the shaft 60 is an elongate cross shape in contrast with the solid elongate cylindrical tube shape as shown in FIG. 3.

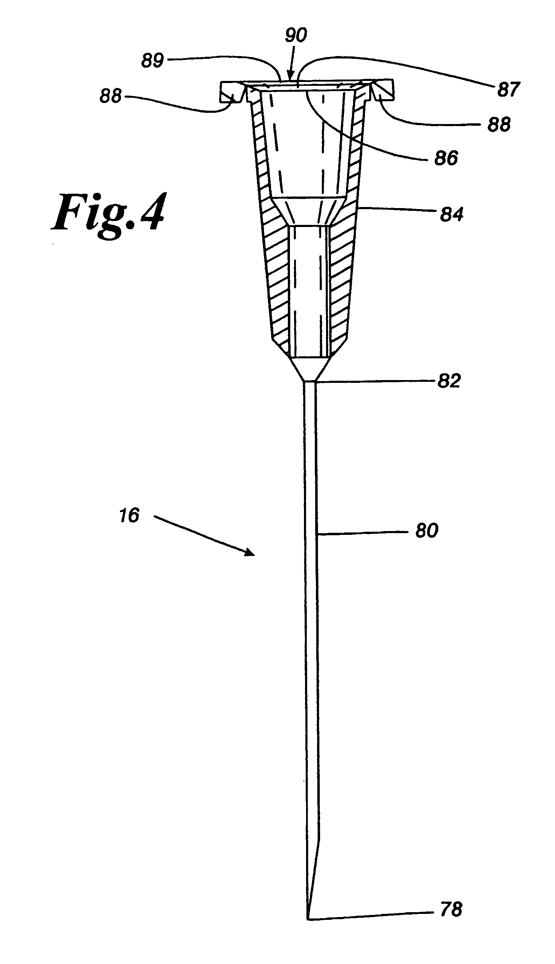

FIG. 4 is a cross sectional view of the needle body 16 shown in FIG. 1. The needle body 16 includes a hollow capillary channel 80 having the previously mentioned piercing tip end 78 and a ferrule end 82 opposing the piercing tip end 78, and the previously mentioned ferrule 84 attached to the ferrule end 82 of the hollow capillary channel 80. The ferrule 84 has a foundation 86 and a grommet 88. The foundation 86 has a surface 87. The grommet 88 has a surface 89 that is temporarily and peripherally attached to the surface 87 of the foundation 86 of the ferrule 84.

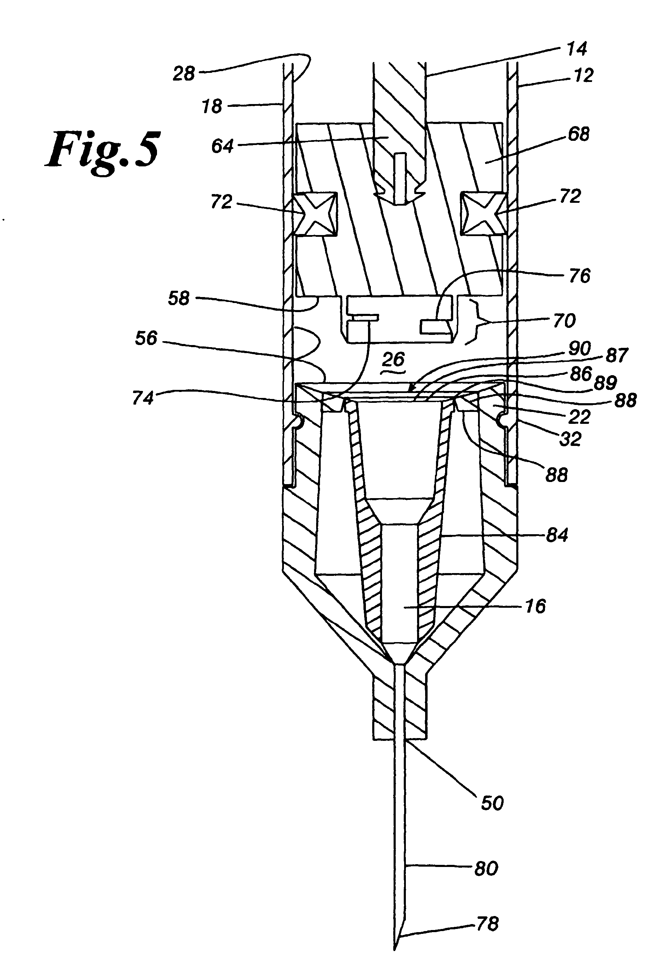

FIG. 5 is a detailed enlarged cross sectional view of a portion of the safety syringe apparatus 10 shown in FIG. 1. The needle body 16 is temporarily attached to the needle attachment 22 of the syringe body 12. In a preferred embodiment, the surface 89 of the grommet 88 temporarily and peripherally attaches the surface 87 of the foundation 86 of the ferrule 84 to the needle attachment 22 of the syringe body 12 to form a surface 90 that is slightly concave. In this alternative embodiment, the resulting surface 90 may otherwise be concave or substantially flat. In an alternative embodiment, the surface 89 of the grommet 88 temporarily and peripherally attaches the surface 87 of the foundation 86 of the ferrule 84 to the inner surface 28 of the tube 18 to form a surface 90 that is slightly concave. In this alternative embodiment, the resulting surface 90 may otherwise be concave or substantially flat.

The tube 18 of the syringe body 12 also has a fluid compartment 26 defined by a stationary surface 56 and a surface 58. The stationary surface 56 of the fluid compartment 26 includes the slightly concave surface 90 and the inner surface 28 of the tube 18 extending within and along the tube 18 between the needle attachment 22 and the surface 58. The surface 58 of the fluid compartment 26 includes the piston 68. The piston 68 is slidably disposed, via the piston seal 72, against the inner surface 28 of the tube 18 while the plunger 14 is displaced into and out of the syringe body 12.

The punch 70 is attached to the piston end 64, positioned adjacent the piston 68 and protrudes toward the needle attachment 22 when the apparatus 10 is assembled. The punch 70 is a substantially hollow cylinder. In a preferred embodiment, the punch 70 is equipped with an upper proximal block tab 74 extending around less than about one-half of the circumference of the substantially hollow cylinder, and a lower distal wedge tab 76 extending around less than about one-half of the circumference of the substantially hollow cylinder and located opposite the upper block tab 74. The hollow capillary channel 80 and the piercing tip end 78 of the needle body 16 are slidably disposed within the needle receiving conduit 50 of the needle attachment 22.

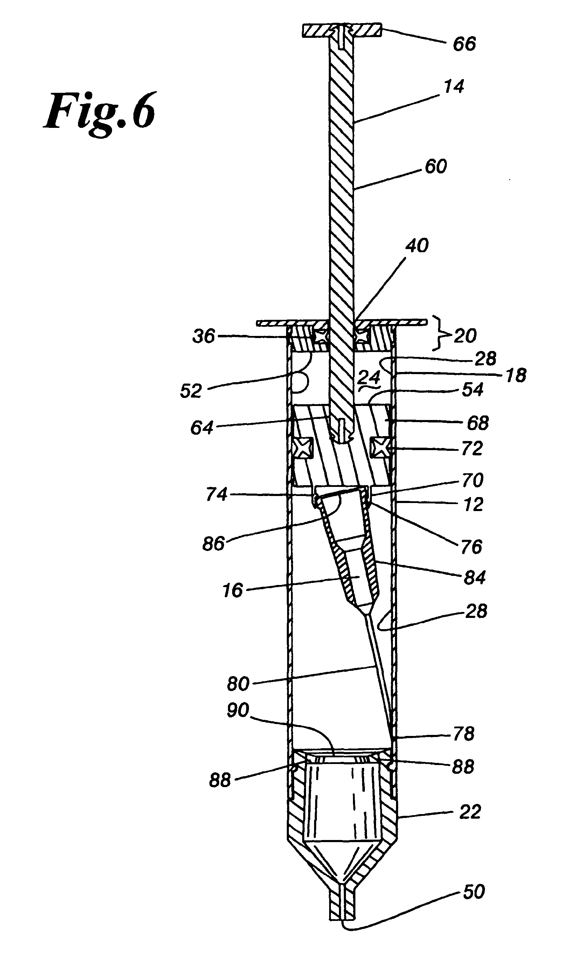

FIG. 6 is a cross sectional view of the needle body 16 permanently residing enclosed within the syringe body 12 and protectively pressed against the inner surface 28 of the syringe body 12 immediately after a single use in accordance with the present invention. The punch 70 frictionally engages the foundation 86 of the ferrule 84, with the upper block tab 74 and the lower wedge tab 76, and the needle body 16 permanently resides enclosed within the syringe body 12 and protectively pressed against the inner surface 28 of the syringe body 12 automatically and immediately after an injection stroke of a single use. The variable vacuum compartment 24 of the syringe body 12 is defined by a stationary surface 52 and a piston surface 54 of the piston 68. The stationary surface 52 of the variable vacuum compartment 24 includes the shaft seal attachment 20 and the inner surface 28 of the tube 18 extending within and along the tube 18 between the shaft seal attachment 20 and the piston surface 54. The shaft 60 is positioned within the shaft orifice 40 of the shaft seal attachment 20 and is slidably engageable with the shaft seal 36. The diameter of the shaft 60, is small enough to provide sufficient volume within the variable vacuum compartment 24 necessary for creating a vacuum, while large enough to provide the requisite structural integrity to the shaft 60. The piercing tip end 78 of the needle body 16 as well as the hollow capillary channel 80 of the needle body 16 are slidably disposed within the needle receiving conduit 50 of the needle attachment 22.

In operation, as shown in FIG. 5 and FIG. 6, during an injection stroke of a single injection of fluid, a vacuum is created within the variable vacuum compartment 24 as a result of an increase in volume within the variable vacuum compartment 24 without a corresponding influx of air molecules, due to the shaft seal 36 and the piston seal 72 creating and maintaining a substantially air tight and fluid tight seal between not only the shaft 60 and the shaft seal attachment 20, but also the piston 68 and the inner surface 28 of the tube 18. At the end of the injection stroke, the upper block tab 74 and the lower wedge tab 76 of the punch 70 frictionally engage the foundation 86 of the ferrule 84. The needle body 16 is immediately and automatically withdrawn within the syringe body 12 as a result of the vacuum created within the variable vacuum compartment 24 during the injection stroke. The needle body 16 is withdrawn into the syringe body 12 such that the piercing tip end 78 permanently resides enclosed within and protectively pressed against the tube 18. This alleviates needle reuse and accidental needle prickings and, therefore, ultimately prevents the transmission of blood-borne pathogens and other diseases by contaminated syringe needles.

More specifically, as shown in FIG. 5 and FIG. 6, to effectuate an intake of fluid into the fluid compartment 26 of the syringe body 12, the thumb platform 66 of the plunger 14 should first be depressed by thrusting the thumb platform 66 partially towards the slightly concave surface 90 to remove a majority of the air present within the fluid compartment 26. During this depression, the piston 68 of the plunger 14 is forced to slide, via the piston seal 72 of the piston 68, against the inner surface 28 of the tube 18 partially towards the slightly concave surface 90. Because the shaft seal 36 of the shaft seal attachment 20 and the piston seal 72 of the piston 68 are substantially air tight and fluid tight, a corresponding influx of air molecules and fluid molecules into the variable vacuum compartment 24 of the syringe body 12 is prevented. As a result, the volume within the variable vacuum compartment 24 is increased without a corresponding influx of air molecules or fluid molecules therefore creating a vacuum within the variable vacuum compartment 24. Secondly, while the thumb platform 66 remains forceably depressed, the piercing tip end 78 of the needle body 16 is submerged into a fluid present within a fluid container. Once the piercing tip end 78 is submerged within the fluid, the thumb platform 66 is allowed to move, as a result of the vacuum created within the variable vacuum compartment 24, away from the needle attachment 22 thus effectuating a withdraw of a desired amount of fluid from a fluid container into the fluid compartment 26 of the syringe body 12.

Now that the fluid is located within the syringe body 12, any residual air must be removed prior to injection. By inverting the safety syringe apparatus so that the piercing tip end 78 is substantially vertical, the fluid, which is more dense than the residual air, is pulled by gravity towards the piston 68 of the plunger 14 while the residual air rises away from the piston 68 towards the slightly concave surface 90. While maintaining the safety syringe apparatus in this substantially vertical position the thumb platform 66 of the plunger 14 is slightly depressed by thrusting the thumb platform 66 only partially towards the slightly concave surface 90, just enough to remove all of the residual air present within the syringe body 12.

As shown in FIG. 5 and FIG. 6, during an injection stroke of a single injection of fluid, the thumb platform 66 of the plunger 14 is depressed by thrusting the thumb platform 66 towards the slightly concave surface 90 to remove substantially all of the fluid contained within the syringe body 12. During this depression, a vacuum is created, as previously discussed herein above, within the variable vacuum compartment 24. At the end of the injection stroke, the punch 70 of the plunger 14 penetrates the slightly concave surface 90, severs the surface 89 of the grommet 88 of the ferrule 84 and frictionally engages the foundation 86 of the ferrule 84 with the upper block tab 74 and lower wedge tab 76 of the punch 70. The foundation 86 of the ferrule 84, which is now frictionally engaged by the punch 70, is immediately and automatically retracted into the syringe body 12 as a result of the vacuum created within the variable vacuum compartment 24. This causes the piercing tip end 78 of the needle body 16 to permanently reside enclosed within the syringe body 12 and protectively pressed against the inner surface 28 of the tube 18 automatically and immediately after a single injection of fluid. Thus, the invented syringe apparatus 10 prevents needle reuse and accidental needle prickings and therefore ultimately alleviates the transmission of blood-borne pathogens and other diseases by contaminated syringe needles.

In one embodiment as shown in FIG. 5 and FIG. 6, the automatically retractable needle safety syringe apparatus 10 has a syringe body 12 that is transparent to allow visibility of the injection fluid within the fluid compartment 26. The safety syringe apparatus 10 has a capacity for injecting at least about 1cc of fluid, and preferably from about 3cc to about 10cc of fluid, to create the requisite amount of vacuum within the variable vacuum compartment 24 for withdrawing the needle body 16 into the syringe body 12. The piercing tip end 78 then permanently resides enclosed within the syringe body 12 and protectively pressed against the inner surface 28 of the tube 18.

In an alternative embodiment, the piercing tip end 78 permanently and protectively resides enclosed within the fluid compartment 26 of the syringe body 12, without being pressed against the inner surface 28 of the tube 18, automatically and immediately after a single injection of fluid.

### SUMMARY OF THE ACHIEVEMENT OF THE OBJECTS OF THE INVENTION

From the foregoing, it is readily apparent that I have invented an automatically retractable needle safety syringe apparatus that provides for injecting fluid from a syringe and subsequently automatically and immediately retracting the piercing tip end of a hollow needle permanently within the safety syringe body, protectively pressed against the inner surface of the tube, after a single use thus preventing the transmission of blood-borne pathogens, such as HIV/AIDS, hepatitis B, hepatitis C and other diseases, via needle reuse and accidental needle prickings with contaminated syringe needles. It is also readily apparent that I have invented an automatically retractable needle safety syringe apparatus that provides for a method of automatically and immediately retracting the piercing tip end of a hollow needle permanently within the safety syringe body, protectively pressed against the inner surface of the tube, after a single use thus preventing the transmission of blood-borne pathogens, such as HIV/AIDS, hepatitis B, hepatitis C and other diseases, via needle reuse and accidental needle prickings with contaminated syringe needles.

The present invention also provides a safety syringe apparatus that permanently prevents needle reuse or accidental needle prickings with contaminated syringe needles by providing a protective barrier between the hands and the hollow needle automatically and immediately after a single use and during disposal. The present invention also provides an automatically and immediately retractable needle safety syringe apparatus that is both efficient and effective at preventing the transmission of blood-borne pathogens and other diseases through needle reuse or accidental needle prickings with contaminated syringe needles. The present invention also provides an automatically and immediately retractable needle safety syringe apparatus that is simple and inexpensive to manufacture, relative to the cost of manufacturing standard or conventional hypodermic needle syringes. The present invention also provides an automatically and immediately retractable needle safety syringe apparatus that is simple and self evident to operate and therefore requires no additional training to use the apparatus effectively and efficiently. The present invention also provides an automatically and immediately retractable needle safety syringe apparatus having a safety feature that is an integral part of the safety syringe, meaning that the safety feature is built-in as a necessary, inherent and integral part of the apparatus and is therefore not merely an accessory which might be removed, circumvented or overridden.

It is to be understood that the foregoing description and specific embodiments are merely illustrative of the best mode of the invention and the principles thereof, and that various modifications and additions may be made to the apparatus by those skilled in the art, without departing from the scope of this invention which is intended to be limited only by the scope of the appended claims.

## Claims

1. An automatically retractable needle safety syringe apparatus (10), comprising:
a syringe body (12) having seal means (36) on an end (30) thereof;
a needle body (16) having attachment means (22) for temporarily attaching said needle body to said syringe body (12); and
a plunger (14) having seal means (72) and punch means (70), said seal means of said plunger and said seal means of said syringe body together creating a vacuum within said syringe body, said punch means of said plunger (14) for engaging and withdrawing said needle body (16) within said syringe body (12), **characterized in that**
said punch (70) has at least two internal, longitudinally offset tabs (74, 76) for frictionally engaging said needle body.

2. The safety syringe apparatus according to claim 1, wherein:
said syringe body (12) has a pair of opposing ends (30,32) and a variable vacuum compartment (24) within said syringe body, said variable vacuum compartment (24) located at the first end (30) of said syringe body;
said needle body (16) has a surface temporarily attached to said syringe body (12) at a second end (32) of said syringe body; and
said plunger (14) is slidably engageable with said syringe body (12) having the punch (70) attached to said plunger (14), said punch having a piercing edge (78) for piercing said surface of said needle body (16).

3. A safety syringe apparatus according to claim 1 or 2, wherein said pair of internal longitudinally offset tabs (74, 76) of said punch (70) comprise:
a first internal proximal tab (74) shaped to press against said needle body (16) at the end of the injection stroke; and
a second internal distal tab (76) shaped to engage said needle body (16) at the end of the injection stroke.

4. A safety syringe apparatus according to claim 3, wherein said syringe body (12) further comprises:
a shaft seal attachment end (30);
a needle attachment end (32) opposing said shaft seal attachment end (30);
and a shaft seal (36) insertably attachable into said shaft seal attachment end (30).

5. A safety syringe apparatus according to claim 4, wherein said needle body (16) is temporarily attached to said needle attachment end (32) of said syringe body (12), said needle body further comprises:
a ferrule (84); and
a piercing tip end (78) attached to said ferrule (84).

6. A safety syringe apparatus according to claim 5, wherein said plunger (14), further comprises:
a shaft (60) having a piston (68) attached thereto, said shaft slidably engageable with said shaft seal (36) of said syringe body (12);
a piston seal (72) attached to said piston and slidably disposed within said syringe body (12); and
a punch (70) attached to said piston (68).

7. A safety syringe apparatus according to claim 6, wherein said syringe body (12) between said shaft seal (36) and said piston seal (72) defines said variable vacuum compartment (24).

8. A safety syringe apparatus according to claim 7, wherein said syringe body (12) further comprises a tube (18), a shaft seal attachment (20) and a needle attachment (22), said tube of said syringe body (12) having a shaft seal attachment end (30), a needle attachment end (32) opposing said shaft seal attachment end (30) and an inner surface extending within and along said tube (18) between said shaft seal attachment end of said tube and said needle attachment end (32) of said tube (18), said shaft seal attachment (20) having said shaft seal (36) interposed therein, said needle attachment (22) having a needle receiving conduit (50), said shaft seal attachment (20) and said shaft seal (36) together insertably attachable to said shaft seal attachment end (30) of said tube (18), said needle attachment (22) insertably attachable to said needle attachment end (32) of said tube.

9. A safety syringe apparatus according to claim 8, wherein said plunger (14) further comprises a thumb end (62) and a piston end (64) opposing said thumb end (62), a shaft (60) slidably engageable with said shaft seal (36) of said shaft seal attachment (20), a thumb platform (66) attached to said thumb end (62), a piston (68) attached to said shaft (60), and a punch (70) attached to said piston (68), said punch (70) positioned adjacent said piston (68) and protruding toward said needle attachment (22).

10. A safety syringe apparatus according to claim 9, wherein said piston (68) further comprises a piston seal (72) attached to said plunger (14), said piston (68) slidably disposed, via said piston seal (72), against said inner surface (28) of said tube (18); preferably:
- said piston (68) has a piston surface (54);
- said shaft seal attachment (20) and said inner surface (28) of said tube (18) extending within and along said tube (18) between said shaft seal attachment (20) and said piston surface (54) define a stationary surface (52); and
- said stationary surface (52) and said piston surface (54) define said variable vacuum compartment (24).

11. A safety syringe apparatus according to claim 9, wherein said needle body (16) further comprises a hollow capillary channel (80), said hollow capillary channel having a piercing tip end (78) and a ferrule (84) end opposing said piercing tip end said ferrule attached to said ferrule (84) end of said hollow capillary channel (80), said piercing tip end (78) and said hollow capillary channel (80) slidably disposed within said needle receiving conduit (50) of said needle attachment (22), said ferrule (84) having a foundation (86) and a grommet (88), said foundation having a surface (87), said grommet having a surface (89) temporarily and peripherally attached to said surface (87) of said foundation (86), preferably said surface (89) of said grommet (88) comprises means for temporarily and peripherally attaching said surface (87) of said foundation (86) of said ferrule (84) to said needle attachment (22) of said syringe body (12) forming a slightly concave surface (90).

12. A safety syringe apparatus according to claim 9,
wherein said proximal tab is block shaped; and
wherein said distal tab is wedge shaped, preferably said punch (70) is a substantially hollow cylinder, said proximal block tab (74) extends around less than about one-half of the circumference of said substantially hollow cylinder, and said distal wedge tab (76) extends around less than about one-half of the circumference of said substantially hollow cylinder opposite said proximal block tab.

13. A safety syringe apparatus according to claim 8, wherein said punch (70) is attached to said piston (68) and positioned adjacent said piston end (64) of said plunger (14).

14. A method of permanently and protectively retracting a hollow needle automatically within a safety syringe apparatus (10), said safety syringe apparatus having a syringe body (12), a plunger (14) and a needle body (16), the syringe body (12) having a variable vacuum compartment (24) and an inner surface (28), the plunger (14) having a punch (70), said punch having internal, longitudinally offset tabs (74,76), the needle body (16) having a ferrule (84) and a piercing tip end (78), the ferrule (84) of the needle body (16) having a grommet (88) and a foundation (86), comprising the steps of:
creating a vacuum within the variable vacuum compartment (24) of the syringe body (12);
severing the grommet (88) of the ferrule (84) with the punch (70) of the plunger; (14) frictionally engaging the foundation (86) of the ferrule (84);
automatically retracting the piercing tip end (78) of the needle body (16) to a position within the syringe body (12), as a result of the vacuum created within the variable vacuum compartment (24);
permanently enclosing the piercing tip end (78) of the needle body (16) within the syringe body (12); and
protectively pressing the piercing tip end of the needle body (16) against the inner surface (28)of the syringe body (12).

## Patentansprüche

1. Sicherheitsspritzensystem mit automatisch zurückziehbarer Kanüle (10), umfassend:
einen Spritzenkörper (12) mit an einem Ende (30) davon befindlichen Dichtungselement (36); einen Kanülenkörper (16) mit Befestigungselement (22) für die temporäre Befestigung des Kanülenkörpers an genanntem Spritzenkörper (12); und
einen Kolben (14) mit Dichtungselement (72) und Stempelelement (70); genanntes Dichtungselement des genannten Kolbens und genanntes Dichtungselement des genannten Spritzenkörpers zusammen ein Vakuum innerhalb des genannten Spritzenkörpers bildend;
genanntes Stempelelement des genannten Kolbens (14) für das Halten und Zurückziehen des Kanülenkörpers (16) innerhalb des genannten Spritzenkörpers (12), **dadurch** charakterisiert, dass
genannter Stempel (70) mindestens zwei innere, longitudinal versetzte Nasen (74,76) für das kraftschlüssige Halten des Kanülenkörpers aufweist.

2. Sicherheitsspritzensystem gemäß Anspruch 1, worin:
genannter Spritzenkörper (12) ein Paar sich gegenüberliegende Enden (30,32) und einen variablen Vakuumabschnitt (24) innerhalb des genannten Spritzenkörpers aufweist, mit genanntem variablem Vakuumabschnitt (24) angeordnet am ersten Ende (30) des genannten Spritzenkörpers;
genannter Kanülenkörper (16) eine Oberfläche für die temporäre Befestigung an genanntem Spritzenkörper (12) am zweiten Ende (32) des genannten Spritzenkörpers aufweist; und genannter Kolben (14) mit dem Stempel (70), befestigt an dem genannten Kolben (14), gleitend durch den genannten Spritzenkörper (12) geführt wird; genannter Stempel eine scharfe Kante (78) zum Durchdringen der genannten Oberfläche des genannten Kanülenkörpers (16) besitzt.

3. Sicherheitsspritzensystem gemäß Anspruch 1 oder 2, worin das genannte Paar der inneren longitudinal versetzten Nasen (74, 76) des genannten Stempels (70) umfasst:
eine erste inneren proximale Nase (74), so geformt, dass sie sich gegen den genannten Kanülenkörper (16) am Ende des Einspritzhubs drückt; und
eine zweite innere distale Nase (76), so geformt, dass sie den genannten Kanülenkörper (16) am Ende des Einspritzhubs hält.

4. Sicherheitsspritzensystem gemäß Anspruch 3, worin genannter Spritzenkörper (12) des Weiteren umfasst:
ein Ende mit Schaftdichtungsbefestigung (30);
ein Ende mit Kanülenbefestigung (32) gegenüberliegend dem Ende mit der Schaftdichtungsbefestigung (30);
und eine Schaftdichtung (36), die durch Einführen in das Ende mit der Schaftdichtungsbefestigung (30) positionierbar ist ;

5. Sicherheitsspritzensystem gemäß Anspruch 4, worin genannter Kanülenkörper (16) temporär an dem genannten Ende mit der Kanülenbefestigung (32) des genannten Spritzenkörpers (12) befestigt ist und genannter Kanülenkörper weiterhin umfasst:
eine Hülse (84) und
ein scharfes Spitzenende (78), welches an genannter Hülse (84) befestigt ist.

6. Sicherheitsspritzensystem gemäß Anspruch 5, worin genannter Kolben (14) des Weiteren umfasst:
einen Schaft (60) mit einem daran befestigten Plunger (68); genannter Schaft wird gleitend durch genannte Schaftdichtung (36) des genannten Spritzenkörpers (12) geführt;
eine Plungerdichtung (72) befestigt an genanntem Plunger und gleitend beweglich innerhalb des genannten Spritzenkörpers (12); und
einen Stempel (70) befestigt an genanntem Plunger (68).

7. Sicherheitsspritzensystem gemäß Anspruch 6, worin genannter Spritzenkörper (12) zwischen der genannten Schaftdichtung (36) und der genannten Plungerdichtung (72) genannten variablen Vakuumabschnitt (24) bildet.

8. Sicherheitsspritzensystem gemäß Anspruch 7, worin genannter Spritzenkörper (12) des Weiteren einen Hohlkörper (18), eine Schaftdichtungsbefestigung (20) und eine Kanülenbefestigung' (22), wobei genannter Hohlkörper des genannten Spritzenkörpers (12) ein Ende mit Schaftdichtungsbefestigung (30) aufweist; ein Ende mit Kanülenbefestigung (32) gegenüberliegend dem Ende mit Schaftdichtungsbefestigung (30) und eine innere Oberfläche, welche sich innerhalb und entlang des genannten Hohlkörpers (18) zwischen genanntem Ende mit Schaftdichtungsbefestigung des genannten Hohlkörpers und genanntem Ende mit Kanülenbefestigung (32) des genannten Hohlkörpers(18) erstreckt, genannte Schaftdichtungsbefestigung (20) aufweisend dazwischen liegende genannte Schaftdichtung (36), genannte Kanülenbefestigung (22) mit kanülenaufnehmender Führung (50), genannte Schaftdichtungsbefestigung (20) und genannte Schaftdichtung (36) zusammen durch Einführen an genanntem Ende mit Schaftdichtungsbefestigung (30) des genannten Hohlkörpers (18) befestigbar, genannte Kanülenbefestigung (22) durch Einführen an genanntem Ende mit Kanülenbefestigung (32) des genannten Hohlkörpers anordbar, umfasst.

9. Sicherheitsspritzensystem gemäß Anspruch 8, worin genannter Kolben (14) weiterhin umfasst ein Kolbenende (62) und ein Plungerende (64) gegenüberliegend dem genannten Kolbenende (62), einen Schaft (60) gleitend geführt durch genannte Schaftdichtung (36) von genannter Schaftdichtungsbefestigung (20), eine Druckfläche (66), befestigt an genanntem Kolbenende (62), einen Plunger (68), befestigt an genanntem Schaft (60) und einen Stempel (70), befestigt an genanntem Plunger (68), genannter Stempel (70) positioniert angrenzend an genanntem Plunger (68) und hervordringend in Richtung zur genannten Kanütenbefestigung (22).

10. Sicherheitsspritzensystem gemäß Anspruch 9, worin genannter Plunger (68) im Weiteren eine Plungerdichtung (72), befestigt an genanntem Kolben (14), wobei genannter Plunger (68) gleitend durch genannte Plungerdichtung (72) gegen genannte innere Oberfläche (28) des genannten Hohlkörpers (18) beweglich ist, umfasst; vorzugsweise
- besüzt genannter Plunger (68) eine Plungeroberfläche (54);
- bilden genannte Schaftsdichtungsbefestigung (20) und genannte innere Oberfläche (28) des genannten Hohlkörpers (18), die sich innerhalb und entlang des genannten Hohlkörpers (18) zwischen genannter Schaftdichtungsbefestigung (20) und genannter Plungeroberfläche (54) erstreckt, eine unveränderliche Oberfläche (52); und
- genannte unveränderliche Oberfläche (52) und genannte Plungeroberfläche (54) bilden genannten variablen Vakuumabschnitt (24).

11. Sicherheitssprüzensystem gemäß Anspruch 9, worin genannter Kanülenkörper (16) des Weiteren eine Hohlkapillare (80) umfasst, wobei genannte Hohlkapillare ein scharfes Spitzenende (78) und ein Ende mit einer Hülse (84) gegenüberliegend dem scharfen Spitzenende aufweist, und genannte Hülse an genanntem Ende der Hülse (84) der genannten Hohlkapillare (80) befestigt ist, sowie genanntes scharfes Spitzenende (78) und genannte Hohlkapillare (80), gleitend beweglich innerhalb genannter kanülenaufnehmender ' Führung (50) der genannten Kanülenbefestigung (22) angebracht, genannte Hülse (84) mit einer Basis (86) und einem Dichtkörper (88), genannte Basis mit einer Oberfläche (87), genannten Dichtkörper mit einer Oberfläche (89), temporär und peripher befestigbar an genannter Oberfläche (87) der genannten Basis (86), genannte Oberfläche (89) des genannten Dichtkörpers (88) vorzugsweise aufweisend Elemente für die temporäre und periphere Befestigung genannter Oberfläche (87) der genannten Basis (86) der genannten Hülse (84) an genannter Kanülenbefestigung (22) des genannten Spritzenkörpers (12), welche eine leicht konkave Oberfläche (90) bilden.

12. Sicherheitsspritzensystem gemäß Anspruch 9, wobei genannte proximale Nase wie ein Block geformt ist; und worin genannte distale Nase wie ein Keil geformt ist; genannter Stempel (70) vorzugsweise im Wesentlichen einen Hohlzylincier darstellt, genannte proximale blockförmige Nase (74) sich um etwas weniger als die Hälfte des Umfangs des genannten weitestgehenden Hohlzylinders erstreckt und genannte keilförmige Nase (76) sich um weniger als die Hälfte des Umfangs des genannten weitestgehenden Hohlzylinders gegenüber der genannten proximalen blockförmigen Nase erstreckt.

13. Sicherheitsspritzensystem gemäß Anspruch 8, worin genannter Stempel (70) an genanntem Plunger (68) befestigt und nahe dem genannten Plungerende (64) des genannten Kolbens (14) positioniert ist.

14. Methode für automatisches dauerhaftes und sicheres Zurückziehen einer Hohlkanüle in einem Sicherheitsspritzensystem (10); genanntes Sicherheitsspritzensystem aufweisend einen Spritzenkörper (12), einen Kolben (14) und einen Kanülenkörper (16), den Spritzenkörper (12) mit einem variablen Vakuumabschnitt (24) und einer inneren Oberfläche (28), den Kolben (14) mit einem Stempel (70), genannten Stempel mit inneren, longitudinal versetzten Nasen (74,76), den Kanülenkörper (16) mit einer Hülse (84) und einem scharfen Spitzenende (78), die Hülse (84) des Kanülenkörpers (16) mit einem Dichtkörper (88) und einer Basis (86), umfassend die folgenden Schritte:
Bildung eines Vakuums innerhalb des variablen Vakuumabschnitts (24) des Spritzenkörpers
(12);
Trennung von Dichtkörper (88) der Hülse (84) und Stempel (70) des Kolbens (14); kraftschlüssiges Halten der Basis (88) der Hülse (84).
Automatisches Zurückziehen des scharfen Spitzenendes (78) des Kanülenkörpers (16) in eine Position innerhalb des Spritzenkörpers (12), infolge des innerhalb des Vakuumabschnitts (24) gebildeten Vakuums;
Dauerhaftes Umschließen des scharfen Spitzenendes (78) des Kanülenkörpers (16) innerhalb des Spritzenkörpers (12); und
sicheres Drücken des scharfen Spüzenendes des Kanülenkörpers (16) gegen die innere Oberfläche (28) des Spritzenkörpers (12).

## Revendications

1. Dispositif formant seringue de sécurité (10) à aiguille automatiquement rétractable comprenant:
un corps de seringue (12) ayant un moyen d'étanchéité (36) à l'une de ses extrémités (30) ;
un corps d'aiguille (16) ayant un moyen de fixation (22) pour fixer temporairement ledit corps d'aiguille audit corps de seringue (12) ; et
un plongeur (14) ayant un moyen d'étanchéité (72) et un moyen de perforation (70), ledit moyen d'étanchéité dudit plongeur et ledit moyen d'étanchéité dudit corps de seringue créant ensemble un vide dans ledit corps de seringue, ledit moyen de perforation dudit plongeur (14) étant prévu pour une mise en prise et un retrait dudit corps d'aiguille (16) dans ledit corps de seringue (12), **caractérisé en ce que**
ledit perforateur (70) possède au moins deux pattes internes longitudinalement décalées (74, 76) pour une mise en prise de friction avec ledit corps d'aiguille.

2. Dispositif formant seringue de sécurité selon la revendication 1, dans lequel :
ledit corps de seringue (12) possède deux extrémités opposées (30, 32) et un compartiment à vide variable (24) dans ledit corps de seringue, ledit compartiment à vide variable (24) étant situé à la première extrémité (30) dudit corps de seringue ;
ledit corps d'aiguille (16) possède une surface temporairement fixée audit corps de seringue (12) à une seconde extrémité (32) dudit corps de seringue ; et
ledit plongeur (14) peut être mis en prise d'une manière coulissante avec ledit corps de seringue (12) ayant le perforateur (70) attaché audit plongeur (14), ledit perforateur ayant un bord perçant (78) pour transpercer ladite surface dudit corps d'aiguille (16).

3. Dispositif formant seringue de sécurité selon la revendication 1 ou 2, dans lequel les deux pattes internes longitudinalement décalées (74, 76) dudit perforateur (70) comprennent :
une première patte proximale interne (74) configurée pour exercer une pression sur ledit corps d'aiguille (16) à la fin de la course d'injection ; et
une deuxième patte distale interne (76) configurée pour venir en prise avec ledit corps d'aiguille (16) à la fin de la course d'injection.

4. Dispositif formant seringue de sécurité selon la revendication 3, où ledit corps de seringue (12) comprend en outre :
une extrémité de fixation (30) de joint d'étanchéité d'arbre ;
une extrémité d'attachement d'aguille (32) opposée à ladite extrémité d'attachement de joint d'étanchéité d'arbre (30) ;
et un joint d'étanchéité d'arbre (36) fixé de manière insérable dans ladite extrémité d'attachement de joint d'étanchéité d'arbre (30).

5. Dispositif formant seringue de sécurité selon la revendication 4, où ledit corps d'aiguille (16) est fixé temporairement à ladite extrémité de fixation d'aiguille (32) dudit corps de seringue (12), ledit corps d'aiguille comprenant en outre :
une ferrure annulaire (84) ; et
une extrémité de perforation en pointe (78) fixée à ladite ferrure (84).

6. Dispositif formant seringue de sécurité selon la revendication 5, où ledit plongeur (14) comprend en outre :
un arbre (60) ayant un piston (68) fixé à celui-ci, ledit arbre pouvant être mis en prise de coulissement avec ledit joint d'étanchéité d'arbre (36) dudit corps de seringue (12) ;
un joint d'étanchéité de piston (72) fixé audit piston, et disposé d'une manière coulissante dans ledit corps de seringue (12) ; et
un perforateur (70) fixé audit piston (68).

7. Dispositif formant seringue de sécurité selon la revendication 6, où ledit corps de seringue (12) entre ledit joint d'étanchéité d'arbre (36) et ledit joint d'étanchéité de piston (72) définit ledit compartiment à vide variable (24).

8. Dispositif formant seringue de sécurité selon la revendication 7, où ledit corps de seringue (12) comprend en outre un tube (18), une fixation de joint d'étanchéité d'arbre (20) et une fixation d'aiguille (22), ledit tube dudit corps de seringue (12) ayant une extrémité de fixation de joint d'étanchéité d'arbre (30), une extrémité de fixation d'aiguille (32) opposée à ladite extrémité de fixation de joint d'étanchéité d'arbre (30) et une surface interne s'étendant dans et le long dudit tube (18) entre ladite extrémité de fixation de joint d'étanchéité d'arbre dudit tube et ladite extrémité de fixation d'aiguille (32) dudit tube (18), ladite fixation de joint d'étanchéité d'arbre (20) ayant ledit joint d'étanchéité d'arbre (36) interposé dans celle-ci, ladite fixation d'aiguille (22) ayant un conduit de réception d'aiguille (50), ladite fixation de joint d'étanchéité d'arbre (20) et ledit joint d'étanchéité d'arbre (36) étant fixés ensemble d'une manière insérable à ladite extrémité de fixation de joint d'étanchéité d'arbre (30) dudit tube (18), ladite fixation d'aiguille (22) pouvant être fixée d'une manière insérable à ladite extrémité de fixation d'aiguille (32) dudit tube.

9. Dispositif formant seringue de sécurité selon la revendication 8, où ledit plongeur (14) comprend en outre une extrémité de pouce (62) et une extrémité de piston (64) opposée à ladite extrémité de pouce (62), un arbre (60) pouvant être mis en prise de coulissement avec ledit joint d'étanchéité d'arbre de ladite fixation de joint d'étanchéité d'arbre (20), une plateforme de pouce (66) fixée à ladite extrémité de pouce (62), un piston (68) fixé audit arbre (60) et un perforateur (70) fixé audit piston (68), ledit perforateur (70) étant positionné d'une manière adjacente audit piston (68) et faisant saillie vers ladite fixation d'aiguille (22).

10. Dispositif formant seringue de sécurité selon la revendication 9, où ledit piston (68) comprend en outre un joint d'étanchéité de piston (72) pouvant être fixé audit plongeur (14), ledit piston (68) étant disposé d'une manière coulissante, par ledit joint d'étanchéité de piston (72), contre ladite surface interne (28) dudit tube (18) ; de préférence :
- ledit piston (68) possède une surface de piston (54) ;
- ladite fixation de joint d'étanchéité d'arbre (20) et ladite surface interne (28) dudit tube (18) s'étendant dans et le long dudit tube (18) entre ladite fixation de joint d'étanchéité d'arbre (20) et ladite surface de piston (54) définissent une surface stationnaire (52) ; et
- ladite surface stationnaire (52) et ladite surface de piston (54) définissent ledit compartiment (24) à vide variable.

11. Dispositif formant seringue de sécurité selon la revendication 9, où ledit corps d'aiguille (16) comprend en outre un canal capillaire creux (80), ledit canal capillaire creux ayant une extrémité de perforation en pointe (78) et une extrémité de ferrure (84) opposée à ladite extrémité de perforation en pointe, ladite ferrure étant fixée à ladite extrémité de ferrure (84) dudit canal capillaire creux (80), ladite extrémité de perforation en pointe (78) et ledit canal capillaire creux (80) étant disposés d'une manière coulissante dans ledit conduit de réception d'aiguille (50) de ladite fixation d'aiguille (22), ladite ferrure (84) ayant une assise (86) et une rondelle (88), ladite assise ayant une surface (87), ladite rondelle ayant une surface (89) fixée temporairement et périphériquement à ladite surface (87) de ladite assise (86), de préférence ladite surface (89) de ladite rondelle (88) comprend un moyen pour fixer temporairement et périphériquement ladite surface (87) de ladite assise (86) de ladite ferrure (84) à ladite fixation d'aiguille (22) dudit corps de seringue (12) formant une surface légèrement concave (90).

12. Dispositif formant seringue de sécurité selon la revendication 9, où ladite patte proximale est en forme de bloc; et
où ladite patte distale est en forme de coin, de préférence ledit perforateur (70) est un cylindre sensiblement creux, ladite patte de bloc proximale (74) s'étend autour d'un peu moins de la moitié de la circonférence dudit cylindre sensiblement creux, et ladite patte de coin distale (76) s'étend autour d'un peu moins de la moitié de la circonférence dudit cylindre sensiblement creux opposé à ladite patte de bloc proximal.

13. Dispositif formant seringue de sécurité selon la revendication 8, où ledit perforateur (70) est fixé audit piston (68) et est positionné d'une manière adjacente à ladite extrémité de piston (64) dudit plongeur (14).

14. Procédé pour retirer d'une manière permanente et avec protection une aiguille creuse automatiquement dans un dispositif formant seringue de sécurité (10), ledit dispositif formant seringue de sécurité ayant un corps de seringue (12), un plongeur (14) et un corps d'aiguille (16), le corps de seringue (12) ayant un compartiment à vide variable (24) et une surface interne (28), le plongeur (14) ayant un perforateur (70), ledit perforateur ayant des pattes internes, longitudinalement décalées (74, 76), le corps d'aiguille (16) ayant une ferrure (84) et une extrémité de perforation en pointe (78), la ferrure (84) du corps d'aiguille (16) ayant une rondelle (88) et une assise (86), comprenant les étapes consistant à :
créer un vide dans le compartiment à vide variable (24) du corps de seringue (12) ; couper la rondelle (88) de la ferrure (84) avec le perforateur (70) du plongeur (14) ; mettre en prise une friction l'assise (86) de la ferrure (84) ;
rétracter automatiquement l'extrémité de perforation en pointe (78) du corps d'aiguille (16) à une position dans le corps de seringue (12), par suite du vide créé dans le compartiment à vide variable (24) ;
renfermer en permanence l'extrémité de perforation en pointe (78) du corps d'aiguille (16) dans le corps de seringue (12) ; et
presser d'une manière protégée l'extrémité de perforation en pointe du corps d'aiguille (16) contre la surface interne (28) du corps de seringue (12).
